# EUROPÄISCHE PATENTSCHRIFT

(11) **EP 0 599 981 B1**
(45) Veröffentlichungstag und Bekanntmachung des Hinweises auf die Patenterteilung: **08.01.1997**
(21) Anmeldenummer: 92918405.9
(22) Anmeldetag: 05.08.1992
(51) Int. Cl.: A61L 2/00, C12Q 1/24

(54) **VERFAHREN ZUR KONTROLLE DER ABREICHERUNGSRATE VON PYROGENEN SUBSTANZEN, INSBESONDERE VIREN, IN ORGANISCHEM MATERIAL**
METHOD OF CHECKING THE RATE OF REMOVAL OF PYROGENIC SUBSTANCES, IN PARTICULAR VIRUSES, FROM ORGANIC MATERIAL
PROCEDE DE CONTROLE DU TAUX D'APPAUVRISSEMENT DE MATERIAUX ORGANIQUES EN SUBSTANCES PYROGENES, NOTAMMENT EN VIRUS

(30) Priorität: 06.08.1991 DE 4126034
(43) Veröffentlichungstag der Anmeldung: 08.06.1994
(73) Patentinhaber: SANORELL PHARMA GmbH & CO., D-72270 Baiersbronn (DE)
(72) Erfinder: BECKER, Gerhard, D-7580 Bühl (DE); LARSON, Marcel, Paul, D-7502 Malsch (DE); HEIDL, Reiner, D-6334 Asslar-Werdorf (DE)
(74) Vertreter: Fritzsche, Thomas M., Dr.
(86) Internationale Anmeldenummer: DE9200653
(87) Internationale Veröffentlichungsnummer: WO9302714

(56) Entgegenhaltungen:
- EP-A- 0 302 949
- WO-A-91/12027

## Beschreibung

Die Erfindung betrifft ein Verfahren zur Kontrolle der Abreicherungsrate von pyrogenen Substanzen, insbesondere von Viren in organischem Material, wobei das zu reinigende Material über einen Ultrafilter oder eine Ultrafiltrationseinheit geleitet wird, deren Abreicherungsrate dadurch bestimmt wurde, indem der Filter oder die Filtrationseinheit mit Viren der Familie Leviviridae oder anderer vergleichbar kleiner Bakteriophagen beaufschlagt wurde und vor und nach der Filtration der Titer der Viren bestimmt und daraus die Abreicherungsrate ermittelt wurde.

Arzneimittel, die aus Zellkulturen, Organen oder Blut von Tier oder Mensch gewonnen werden, sind potentiell mit tier- oder humanpathogenen Viren oder anderem pyrogenen Material kontaminiert. Bei dem breiten Spektrum von Viren, mit denen die Probe verseucht sein kann, ist es unmöglich, das Ausgangsmaterial auf alle Viren, die vorhanden sein könnten, zu testen. Für einige Virusgruppen gibt es außerdem keine zuverlässige oder ausreichend empfindliche Nachweismethode. Deshalb muß ein Reinigungs- oder Inaktivierungsverfahren durchgeführt werden, durch das pathogene Viren abgereichert werden, so daß auch bei einer massiven Infektion von Ausgangsmaterial oder Zwischenprodukten keine Probleme zu erwarten sind. Das Reinigungsverfahren oder Inaktivierungsverfahren soll dabei so effizient sein, daß Viren um Raten bis zu 10¹² abgereichert werden können.

Bei der Herstellung von Arzneimitteln wird seit langem zur Entfernung von Bakterien die Sterilfiltration eingesetzt und gilt als sicheres Dekontaminierungsverfahren für diese potentiellen Pathogene. Die Sterilfilter werden dabei vom Hersteller stichprobenweise mit dem kleinsten, außerhalb der Gruppen der Mykoplasmen und L-Formbakterien bekannten Bakterium Pseudomonas diminuta beaufschlagt. Wenn für dieses Bakterium im "Bakterien-Challenge-Test" eine bestimmte Abreicherungsrate bei der Sterilfiltration nachgewiesen werden kann, gilt die Produktionscharge als sicher. Ein derartiges Verfahren ist beschrieben bei Wallhäußer, Praxis der Sterilisation, Thieme Verlag, Stuttgart, 1988, Seiten 324 ff.

Zur Entfernung von Viren können auch Filtrationsverfahren eingesetzt werden, wobei die verwendeten Filter Moleküle und Partikel über 1 Million Dalton zurückhalten müssen. Solche Ultrafilter sind in verschiedensten Ausführungen verfügbar, jedoch handelt es sich - im Gegensatz zu Sterilfiltern - um keine Absolutfilter; d. h. Moleküle und Partikel über 1 Million Dalton werden nicht absolut, sondern nur zu einem großen Teil zurückgehalten. Dabei ist die Rückhalterate nicht nur abhängig vom Filtertyp, sondern sie kann sich sogar von Produktionscharge zur Produktionscharge unterscheiden. Ultrafilter wurden deshalb bisher noch nicht zur Virusentfernung eingesetzt, sondern trugen allenfalls zur Gesamtvirusabreicherung in einem Produktionsverfahren aus mehreren Schritten bei (Werner und Langlius-Gane, Meeting the Regulatory Requirements for Pharmaceutical Production of Recombinant DNA Derived Products, Arzneimittel-Forschung, Bd. 39 (1989), 108-111). Diese Unzuverlässigkeit von Ultrafiltern ist im Produktionsverfahren der Filter begründet. Bei Ultrafiltern, die für einen definierten Molekulargewichtsausschluß vorgesehen sind, können immer größere Mikroporen auftreten, die z. B. für Viren durchlässig sein können. Außerdem können Ultrafilter nicht wie Mikrofilter durch das sogenannte Bubble-Point-Verfahren auf ihre Dichtigkeit getestet werden.

In der nicht vorveröffentlichten DE-A-40 03 543.3 und der PCT/DE 91/00099, deren Inhalt hiermit ausdrücklich zum Gegenstand der Beschreibung gemacht wird, ist ein Verfahren zur Bestimmung der Abreicherungrate von Viren beschrieben, das auch Auskunft darüber gibt, durch welches Filtrationsverfahren und durch wieviele Filtrationsschritte eine als sicher geltende Abreicherung erreicht wird. Die Abreicherungsrate wird dabei dadurch bestimmt, daß der Filter oder die Filtrationseinheit mit Viren der Familie Leviviridae beaufschlagt wird und vor und nach der Filtration der Titer der Viren bestimmt und daraus die Abreicherungsrate ermittelt wird.

Es ist auch möglich, andere vergleichbar kleine Bakteriophagen zu verwenden. Diese Bakteriophagen sind vorzugsweise durch einfache Löcher in einem Bakterienrasen nachweisbar.

Dieses Verfahren hat jedoch den Nachteil, daß nach der Verwendung eines derart geeichten Filters die Abreicherungsrate nach dem zuvor beschriebenen Verfahren nochmals bestimmt werden muß, um sicherzustellen, daß vor oder während der Filtration keine Beschädigung des Filters, wie beispielsweise das Auftreten von Löchern, Rissen, etc. aufgetreten ist, die ein Rückhalten des abzutrennenden pyrogenen Materials verhindert. Diese zusätzliche Bestimmung der Abreicherungsrate benötigt jedoch einen Aufwand an Zeit und an Material, der die Produktions- und Personalkosten sowie den apparativen Aufwand vergrößert, wenn damit in großtechnischem Maßstab Viren in Lösungen abgereichert werden soll.

Es besteht daher Bedarf an einem schnellen und ohne allzu großen Aufwand auf einfache Weise durchzuführenden Verfahren, mit dem die einmal bestimmte Abreicherungsrate der Filter kontrolliert werden kann.

Dieses Ziel wird nun erfindungsgemäß dadurch erreicht, daß man Verfahren zur Kontrolle der Abreicherungsrate von pyrogenen Substanzen, insbesondere von Viren in organischem Material, das dadurch gekennzeichnet ist, daß das zu reinigende Material über einen Ultrafilter oder eine Ultrafiltrationseinheit geleitet wird, deren Abreicherungsrate vorher dadurch bestimmt wurde, daß der Filter oder die Filtrationseinheit mit Viren der Familie Leviviridae oder anderer vergleichbar kleiner Bakteriophagen beaufschlagt wurde und vor und nach der Filtration den Titer der Viren festgestellt und daraus die Abreicherungsrate ermittelt wurde, und man vor oder nach dem Bestimmen der Abreicherungsrate an den Filter oder die Filtrationseinheit in einem Druckhaltetest mit einem Gas einen vorbestimmten Prüfdruck anlegt, und den Druckabfall über eine vorbestimmte Zeit bestimmt und nach erfolgter Filtration des biologischen Materials die Abreicherungsrate des Filters durch nochmalige Durchführung des Druckhaltetests kontrolliert.

Erfindungsgemäß wurde nämlich gefunden, daß die Abreicherungsrate eines Filters bzw. einer Filtrationseinheit solange konstant ist, solange der Druckabfall in einem Druckhaltetest (einem sog. "forward-flow"-Test) ebenfalls konstant ist. Ändert sich das Verhalten des Filters bei einem solchen Test, dann ist eine Veränderung der Struktur der Mikroporen oder eine mikroskopische Zerstörung der Filterstruktur vorhanden, die ein Zurückhalten der abzureichernden pyrogenen Substanz verhindert und diese ungehindert durch den Filter durchtreten läßt. Damit wird jedoch die Abreicherungsrate des Filters verschlechtert. Erfindungsgemäß wird dabei derart vorgegangen, daß man einen Filter, bei dem die Abreicherungsrate bestimmt wurde, mit seinem dem zu filternden Material zugewandten Seite einem Gas eines vorbestimmten Druckes aussetzt und nach einer Stabilisierungszeit den Druckabfall pro Zeiteinheit bestimmt. Die Stabilisierungszeit ist derart auszuwählen, daß der angelegte Druck sich über sämtliche im System auftretenden Druck verbrauchenden Teilen stabilisiert. Die Höhe des anzuwendenden vorbestimmten Druckes ist vom Fachmann durch einfache Versuche leicht zu ermitteln und hängt von dem jeweils verwendeten Filter und dem Gas ab. Er sollte jedoch unterhalb des Zerstörungsdruckes des Filters liegen, damit die Integrität der Membran nicht durch den Test zerstört wird. Der maximal zulässige Druckabfall ist durch einfache Versuche vom Fachmann ohne weiteres festzustellen und hängt vor dem jeweils verwendeten Filter ab. Nachdem auf diese Weise der für die Abreicherungsrate bei gegebenem Filter charakteristische Druckabfall bestimmt ist, kann nach erfolgter Filtration des biologischen Materials die Abreicherungsrate des Filters allein dadurch kontrolliert werden, daß man mittels eines weiteren Druckhaltetests den Druckabfall bestimmt und diesem mit dem zuvor bestimmten, für die jeweilige Abreicherungsrate charakteristischen Druckabfall vergleicht. Treten keine oder nur geringe Abweichungen im Druckabfall auf, so kann erfindungsgemäß davon ausgegangen werden, daß sich die Abreicherungsrate des Filters durch den Gebrauch nicht verändert hat. Mit dem erfindungsgemäßen Verfahren ist es daher möglich, auf einfache und leicht durchzuführende Weise die Qualität eines validierten Filters ohne großen Aufwand nach jeder Verwendung zu kontrollieren.

In einer bevorzugten Ausführungsform wird der Druckhaltetest an einem Filter durchgeführt, der befeuchtet ist. Bevorzugte Befeuchtungsmittel sind Wasser oder wäßrige Lösungen und Gemische aus Wasser und einem organischen Lösungsmittel. Bevorzugte wäßrige Lösungen sind wäßrige basische Lösungen, insbesondere verdünnte Lösungen von NaOH, KOH und NH₃. Je nach dem zu verwendenden organischen Material sind auch verdünnte Lösungen von schwachen mineralischen oder organischen Säuren als Benetzungsmittel zu verwenden. Bevorzugte organische Lösungsmittel umfassen insbesondere Alkohole und Ketone, wobei Methanol, Ethanol und Propanol sowie Isopropanol bevorzugt sind. Werden die Filter mit den zuvor genannten wäßrigen Lösungen benetzt, so ist darauf zu achten, daß nur solche Lösungen verwendet werden, die mit dem Filtermaterial kompatibel sind.

In einer weiteren bevorzugten erfindungsgemäßen Ausführungsform wird nach Anlegen des Prüfdruckes noch eine gewisse Zeit abgewartet, bis sich der Druckabfall im zu messenden System stabilisiert hat. Die Stabilisierungsdauer hängt vom jeweiligen zu messenden Testsystem ab und ist durch einfache Versuche vom Fachmann ohne weiteres zu ermitteln. Übliche Stabilisierungszeiten betragen bei handelsüblichen Filtern 30 bis 600 s und insbesondere von 200 bis 400 s.

Die Testzeit, in der der Druckabfall bestimmt wird, hängt ebenfalls vom zu testenden System und der gewünschten Genauigkeit ab und ist ebenfalls vom Fachmann ohne weiteres für die gewünschten Bedingungen leicht zu ermitteln. Übliche Testzeiten betragen 15 bis 900 s und liegen vorzugsweise zwischen 200 und 600 s. Zweckmäßigerweise werden Testzeiten von 250 bis 300 s ausgewählt.

Es hat sich als zweckmäßig erwiesen, den zur Kontrolle der Abreicherung nach erfolgter Filtration durchgeführten Druckhaltetest unter genau den gleichen Bedingungen durchzuführen wie vor der Filtration. Es ist jedoch erfindungsgemäß auch möglich, den Kontrolltest unter anderen Bedingungen durchzuführen, wenn sich dies als zweckmäßig erweist und wenn sichergestellt ist, daß der dabei gemessene Druckabfall einer Abreicherungsrate zuordnet werden kann.

Im erfindungsgemäßen Verfahren hat es sich als zweckmäßig erwiesen, für den Prüfdruck als Gas Luft und/oder Edelgase zu verwenden. Besonders bevorzugt sind dabei Stickstoff, Sauerstoff und Wasserstoff, von den Edelgasen wird vorzugsweise Helium verwendet. Für besonders rasch durchzuführende Messungen oder bei Membranen mit besonders kleinen Poren wird vorzugsweise Wasserstoff oder Helium als Prüfdruckgas erwiesen.

Mit dem erfindungsgemäßen Verfahren ist es möglich, eine Ultrafiltration als einzige Methode zur gesicherten Virusabreicherung ohne zusätzliche Reinigungs- oder Inaktivierungsschritte durchzuführen, indem man im laufenden Prozess die Abreicherungsrate der pyrogenen Substanzen kontrolliert. Wenn diese Abreicherungsrate vor und nach der Filtration der Produktionscharge bestimmt wird und höher als 10¹² ist, so kann mit größter Sicherheit eine Virus-Kontamination des Produktes ausgeschlossen werden. Bisher wurden Validierungsversuche nur mit tier- oder sogar mit humanpathogenen Viren durchgeführt. Deshalb konnten aus Sicherheitsgründen die validierten Filter nachher nicht mehr verwendet werden. Aus diesem Grund mußten dann neue Filter mit möglicherweise verschiedenen Abreicherungsraten eingesetzt werden. Eine solche Validierung war daher immer nur für einen einzigen Filter gültig und konnte im übrigen auch wegen des damit verbundenen Aufwands nur einmal vor oder nach einer Abreicherung exemplarisch durchgeführt werden. Im Gegensatz dazu ist es erfindungsgemäß möglich in einem Verfahren die Abreicherung von pyrogenen bzw. pathogenen Substanzen, wie beispielsweise Viren, zu kontollieren.

Erfindungsgemäß wird die zu reinigende Lösung oder auch das zu reinigende Gas über einen Filter oder eine Filtrationseinheit geleitet, deren Abreicherungsrate vorher ermittelt wurde. Durch Einsatz von Viren der Gruppe Leviviridae als Testvirus gelingt es, die Abreicherungsrate sicher nachzuweisen. Durch einen vorherigen oder anschließenden Druckhaltetest wird der so bestimmten Abreicherungsrate einem Druckabfall bei vorbestimmten Bedingungen zugeordnet, solange sich der Druckabfall bei späteren Kontrollmessungen als vergleichbar erweist, kann von einer gleichbleibenden Abreicherungsrate ausgegangen werden.

Die zur Bestimmung der Abreicherungsrate verwendeten Leviviridae sind mit 23 nm Durchmesser und einem Molekulargewicht von 1,4 Millionen Dalton kleiner als tier- oder humanpathogene Viren (H. Fraenkel-Conrat, The Viruses, Catalogue, Characterization and Classification, Plenum Press, 1982). Sie befallen nur Bestimmte F⁺-Stämme des harmlosen Darmbakteriums Escherichia coli und werden als RNA-Viren bereits in 10 mM NaOH innerhalb kurzer Zeit hydrolysiert und dabei in ihre molekularen Einzelbestandteile zerlegt. Die kleinsten tier- und/oder humanpathogenen Viren stammen aus der Gruppe der Picornaviren, sind 27 nm im Durchmesser und 2,5 Millionen Dalton schwer. Damit eignen sich die Leviviridae für die Validierung der nach Größe selektionierenden Ultrafilter. Die Filter können danach mit 0,1 M NaOH gewaschen werden, wodurch auch aus Escherichia coli stammende Pyrogene entfernt werden. Die Filter sind dann wieder für die Produktion einsatzbereit. Die für eine In-Prozess-Kontrolle definierten Bedingungen sind damit erfüllt, nämlich durch:
- ein einfaches, innerhalb kurzer Zeit durchführbares und exaktes Validierungsverfahren
- eine Wiederverwendbarkeit der getesteten Filter ohne zusätzliche Verunreinigung des Produkts.
Außerdem können die Leviviridae in extrem hohen Titern von bis zu 10¹⁴ pfu pro ml angezogen werden und durch ein einfaches Plattierungsverfahren selbst in Konzentrationen von 1 pfu pro ml zuverlässig nachgewiesen werden.

Für die Bestimmung wird der Filter mit einer Viruslösung mit einem Titer von über 10¹⁰ pfu/ml beaufschlagt und die Konzentration der Phagen in Filtrat und in der zurückgehaltenen Lösung bestimmt. Die Bestimmung erfolgt in an sich bekannter Weise (z. B. nach der Top-Agar-Methode von N.H. Adams (1959), Bacteriophages, Interscience Publishers, New York). Dazu werden beispielsweise die Phagen mit geeigneten Wirtsbakterien gemischt (z. B. E. coli 3300, ATCC Nr. 19853) und in einer Schicht von 0,6 % Agar-Agar auf Platten mit Nährstoffagar (z. B. 1 % Bactotrypton, 0,5 % Hefeextrakt, 0,5 % NaCl, 0,1 mM CaCl₂, 1,5 % Agar-Agar) aufgetragen. Pro Platte sollten 10⁷ bis 10⁸ Bakterien und weniger als 100 Phagen aufgetragen werden. Die Platten werden dann bei 37°C inkubiert, worauf nach 10 Stunden ein Bakterienrasen entsteht. Löcher in diesem Rasen zeigen Virusbefall an und die Anzahl der Löcher ergibt den Virustiter in pfu (plaque-forming-units). Da schon ein Virus einen Plaque hervorrufen kann, können auch mit Standardagarplatten 1 Virus pro ml nachgewiesen werden. Es läßt sich also ein Viruskonzentrationsbereich von über 10¹⁴ bis zu 1 pfu pro ml abdecken.

Durch Bestimmung der Viruskonzentration im Filtrat und in der Lösung vor der Filtration ergibt sich dann die Virusabreicherungsrate. Durch Bestimmung des Virustiter im Konzentrat (d. i. die vor dem Filter zurückgehaltene Lösung) kann errechnet werden, ob Viren durch Absorption an den Filter oder Inaktivierung verloren gingen, was dem Validierungsverfahren eine zusätzliche Sicherheit gibt.

Da sich das Abreicherungsverhalten von Filtrationsmembranen nicht nur von Hersteller zu Hersteller, sondern auch von Produktionscharge zu Produktionscharge erheblich unterscheiden kann, ist es wesentlich, die Abreicherungsrate für die pyrogenen Substanzen für jeden individuellen Filter zu bestimmen.

Bevorzugt überprüft man den für die Reinigung des organischen Materials vorgesehenen Filter bzw. die Filteranlage unter genau definierten Druckbedingungen, die dann beim Abreicherungsverfahren später auch eingehalten werden.

Die Filter können dann nach Bestimmung der Abreicherungsrate von den Bakteriophagen und anderen Rückständen wie Pyrogenen durch einfaches Spülen mit Natronlauge entfernt werden und die Filter dann für das Reinigungsverfahren des organischen Materials eingesetzt werden. Dies ist ein weiterer Vorteil des erfindungsgemäßen Verfahrens, da dies bei Verwendung von tier- oder humanpathogenen Viren zur Bestimmung der Abreicherungsrate wegen der großen Gefahr der Kontamination mit derartigen Viren nicht möglich wäre.

Im folgenden wird die Erfindung durch Beispiele näher erläutert.

### Beispiel 1

### Abreicherung der Testviren um den Faktor 12

Getestet wurde die Filtrationspatrone S1Y30 mit der Seriennummer 10330. Die Phagenlösung bestand aus 600 ml Phagenpuffer (Methoden) mit 600 mg Rinderserumalbumin und 50 ml Phagenkonzentrat (Titer: 1,3x10¹² pfu (plaque forming units)/ml). Die Filtration wurde zunächst dreimal hintereinander durchgeführt. Das Volumen des Filtrats verringert sich von 600 über 400 auf 380 ml. Die Filtrationszeiten betrugen je Schritt ca. 20 Minuten. Zwischen jeder Filtration wurde die Patrone zur Inaktivierung von Phagenrückständen mit 1 l 10 mM Natronlauge gewaschen und dann mit dest. Wasser bis zur Neutralität (gemessen mit der pH-Elektrode) gespült.

Der Virusgehalt in den einzelnen Filtraten wurde bestimmt und ist in Tabelle 1 dargestellt.

Aus den Daten der Virusabreicherung (Tabelle 1) geht hervor, daß mit der eingesetzten Ultrafiltrationspatrone nach der ersten Filtration der Virustiter um 6,92 und 7,22 Zehnerlogarithmen abnahm. Nach der jeweils zweiten Filtration waren bereits keine Phagen mehr im Filtrat nachweisbar. Demnach wurden die Viren nach den ersten zwei Filtrationen um insgesamt 11 Zehnerpotenzen und nach den weiteren zwei Filtrationen um 11,7 Zehnerpotenzen reduziert, woraus sich rechnerisch eine Gesamtreduktion um 22,7 Zehnerpotenzen nach vier Filtrationen ergibt. Die in der Literatur häufig empfohlene Abreicherung um 12 bis 16 wird mit 18,22 Zehnerpotenzen bereits nach drei Filtrationen überschritten.

### Beispiel 2

### Überprüfung des Abreicherungsvermögens von Testviren anhand der Druckabfallcharakteristik eines Ultrafilters

Der in Beispiel 1 validierte Filter wird in einem Integrationstestgerät Palltronic FFE-03 (erhältlich durch die Fa. Pall Filtrationstechnik GmbH in D-6072 Dreieich 1) einem nicht destruktiven Druckhaltetest unterzogen. Dabei wurde die gespülte und gewaschene Ultrafiltrationspatrone nacheinander mehreren Druckhaltetests (forward-flow-Tests) unterworfen, die wie folgt durchgeführt wurden:

Die Filtrationspatrone wurde im Testgerät mit 1 bar Stickstoffdruck beaufschlagt. Nach einer Stabilisierungszeit von 300 Sekunden wird der Druckabfall über einen Zeitraum von 300 sec gemessen. Dabei wurden jeweils ein Druckabfalls von 5 mbar, 13 mbar und 10 mbar gemessen. Nach Ende der Messung wird die Patrone automatisch entlüftet und das Meßergebnis ausgedruckt.

### Beispiel 3

Mit der Filtrationspatrone von Beispiel 2 wurde ein Thymusextrakt filtriert.

Der aus der Thymusdrüse auf an sich bekannte Weise erhaltene Extrakt wurde filtriert und die Abnahme an BSA wurde mittels einer HPLC an einer Umkehrphase kontrolliert. Danach wurde die Filtrationspatrone gereinigt und der Druckabfall mittels dem Druckhaltetest wie in Beispiel 2 beschrieben, bestimmt. Dabei wurde bei zwei Messungen ein Druckabfall von 5 und von 14 mbar festgestellt. Dies bedeutet, daß sich die Virusabreicherungsrate nicht verändert hat.

Der Bakteriophage fr ( ATCC 15767-B1 ) wurde am 19. November 1964 bei der American Type Culture Collection, 12301 Parklawn Drive, Rockville, Maryland 20852-1776 USA hinterlegt, und ist seit dem 19. November 1964 frei erhältlich.

E. Coli 3300 (ATCC 19853) wurde am 12. Januar 1967 bei der American Type Culture Collection, 12301 Parklawn Drive, Rockville, Maryland 20852-1776 USA hinterlegt, und ist seit dem 12 Januar 1967 frei erhältlich.

**Tabelle 1**

| Virustiter in den Filtraten der einzelnen Filtrationsschritte mit der Ultrafiltrationspatrone Amicon S1Y30 | |
|---|---|
| Filtrationsschritt | Virustiter (pfu/ml) |
| Ausgangslösung | 1 x 10¹¹ |
| 1. Filtration | 1,2 x 10⁴ |
| 2. Filtration | 0 |
| 3. Filtration | 0 |
| Zugabe neuer Testphagen | 5 x 10¹¹ |
| 1. Filtration | 3 x 10⁴ |
| 2. Filtration | 0 |
| 3. Filtration | 0 |

## Patentansprüche

1. Verfahren zur Kontrolle der Abreicherungsrate von pyrogenen Substanzen, insbesondere von Viren in organischem Material, **dadurch gekennzeichnet**, daß das zu reinigende Material über einen Ultrafilter oder eine Ultrafiltrationseinheit geleitet wird, deren Abreicherungsrate vorher dadurch bestimmt wurde, daß der Filter oder die Filtrationseinheit mit Viren der Familie Leviviridae oder anderer vergleichbar kleiner Bakteriophagen beaufschlagt wurde und vor und nach der Filtration den Titer der Viren festgestellt und daraus die Abreicherungsrate ermittelt wurde, und
man vor oder nach dem Bestimmen der Abreicherungsrate an den Filter oder die Filtrationseinheit in einem Druckhaltetest mit einem Gas einen vorbestimmten Prüfdruck anlegt, und den Druckabfall über eine vorbestimmte Zeit bestimmt und nach erfolgter Filtration des biologischen Materials die Abreicherungsrate des Filters durch nochmalige Durchführung des Druckhaltetests kontrolliert.

2. Verfahren nach Anspruch 1, **dadurch gekennzeichnet**, daß man den Druckabfall an einem Filter bestimmt, der mit Wasser, einer wäßrigen Lösung oder einem Gemisch aus Wasser und einem organischen Lösungsmittel benetzt ist.

3. Verfahren nach Anspruch 2, **dadurch gekennzeichnet**, daß man als wäßrige Lösung eine verdünnte NaOH-, KOH- oder NH₃-Lösung verwendet.

4. Verfahren nach einem der vorhergehenden Ansprüche,
**dadurch gekennzeichnet**,
daß man den Druckabfall erst nach einer Stabilisierungszeit bestimmt.

5. Verfahren nach einem der vorhergehenden Ansprüche,
**dadurch gekennzeichnet**, daß man die
Messung des Druckabfalles nach der Filtration unter den gleichen Bedingungen wie vor der Filtration durchführt.

6. Verfahren nach einem der vorhergehenden Ansprüche,
**dadurch gekennzeichnet**, daß man
als Gas Luft, Stickstoff, Sauerstoff, Wasserstoff oder Helium verwendet.

## Claims

1. Method of checking the removal rate of pyrogenic substances, especially viruses in organic material, characterised in that the material to be purified is passed over an ultrafilter or an ultrafiltration unit, the removal rate of which was previously determined by introducing viruses of the Leviviridae family or other comparably small bacteriophages into the filter or the filtration unit, by ascertaining the titre of the viruses before and after the filtration and by calculating the removal rate therefrom, and, before or after determining the removal rate, a predetermined test pressure is applied to the filter or the filtration unit in a pressurising test using a gas, the decrease in pressure over a predetermined time is determined and, after filtration of the biological material has taken place, the removal rate of the filter is checked by once again carrying out the pressurising test.

2. Method according to claim 1, characterised in that the decrease in pressure is determined at a filter, which is moistened with water, an aqueous solution or a mixture of water and an organic solvent.

3. Method according to claim 2, characterised in that a dilute NaOH, KOH or NH₃ solution is used as the aqueous solution.

4. Method according to one of the preceding claims, characterised in that the decrease in pressure is determined only after a stabilization time.

5. Method according to one of the preceding claims, characterised in that the measurement of the decrease in pressure is effected after the filtration under the same conditions as before the filtration.

6. Method according to one of the preceding claims, characterised in that air, nitrogen, oxygen, hydrogen or helium is used as the gas.

## Revendications

1. Procédé de contrôle du taux d'appauvrissement d'une matière organique en substances pyrogènes, notamment en virus, caractérisé en ce que l'on fait passer la matière à purifier sur un ultrafiltre ou une unité d'ultrafiltration dont on a établi préalablement le taux d'appauvrissement en chargeant le filtre ou l'unité de filtration de virus de la famille des Leviviridae ou d'autres bactériophages de petite taille comparable, en déterminant le titre de virus avant et après la filtration et en établissant ainsi le taux d'appauvrissement, et en ce qu'avant ou après la détermination du taux d'appauvrissement sur le filtre ou l'unité de filtration, on exerce une pression d'essai prédéterminée au moyen d'un gaz dans le cadre d'un essai de tenue de pression et qu'on détermine la chute de pression sur une durée prédéterminée et qu'on contrôle le taux d'appauvrissement du filtre après réalisation de la filtration de la matière biologique par répétition de l'essai de tenue de pression.

2. Procédé selon la revendication 1, caractérisé en ce qu'on détermine la chute de pression sur un filtre mouillé d'eau, d'une solution aqueuse ou d'un mélange d'eau et d'un solvant organique.

3. Procédé selon la revendication 2, caractérisé en ce qu'on utilise comme solution aqueuse une solution diluée de NaOH, KOH ou NH₃.

4. Procédé selon une des revendications précédentes, caractérisé en ce qu'on ne détermine la chute de pression qu'après une période de stabilisation.

5. Procédé selon une des revendications précédentes, caractérisé en ce qu'on réalise la mesure de la chute de pression après la filtration dans les mêmes conditions qu'avant la filtration.

6. Procédé selon une des revendications précédentes, caractérisé en ce qu'on utilise comme gaz de l'air, de l'azote, de l'oxygène, de l'hydrogène ou de l'hélium.
